# EUROPEAN PATENT APPLICATION

(11) **EP 4 706 640 A1**
(43) Date of publication of application: **11.03.2026**
(21) Application number: 25200779.4
(22) Date of filing: 08.09.2025
(51) Int. Cl.: A61K 9/48, A61K 31/495

(54) **CAPSULE FORMULATIONS COMPRISING CARIPRAZINE HYDROCHLORIDE**

(30) Priority: 10.09.2024 TR 2024011968
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); PEHLIVAN AKALIN, Nur, Istanbul (TR); ARMUT, Merve, Istanbul (TR); OZTAS, Bora, Istanbul (TR)
(74) Representative: Genç Ilhan, Oznur

(57) **Abstract**

The present invention relates to a capsule formulation comprising cariprazine hydrochloride and at least one filler and at least one lubricant, wherein the bulk density of this formulation before filling into capsules is at least 0.5 g/cm³ as measured by gravimetric determination of a measured volume. The present invention also relates to a simple, rapid, cost effective, timesaving and industrially convenient method of preparing the formulation.

## Description

### Field of the Invention

The present invention relates to a capsule formulation comprising cariprazine hydrochloride and at least one filler and at least one lubricant, wherein the bulk density of this formulation before filling into capsules is at least 0.5 g/cm³ as measured by gravimetric determination of a measured volume. The present invention also relates to a simple, rapid, cost effective, time-saving and industrially convenient method of preparing the formulation.

### Background of the Invention

Schizophrenia is a lifelong disabling psychiatric disorder with a reported worldwide prevalence of about 1%, including 3.2 million Americans. The disorder usually manifests during adolescence or in young adulthood; the cardinal symptoms fall into three domains - positive symptoms, such as delusions and hallucinations, negative symptoms, such as lack of drive and social withdrawal, and cognitive symptoms, such as problems with attention and memory. These lead to social and occupational dysfunction, which inevitably have a profound effect on the family and the place of the affected individual in wider society. In addition to psychiatric symptoms, patients with schizophrenia are at greater risk for medical comorbidities than the general population.

Drug treatment with dopamine antagonists is the cornerstone of schizophrenia management both during the acute as well as the residual phase. Current guidelines recommend atypical antipsychotics, including risperidone, olanzapine, quetiapine, ziprasidone, and aripiprazole, as first-line treatment for schizophrenia. These drugs can be uniformly characterized by their dual mode of action: in addition to antagonism of the dopamine D2receptor, they are also potent inhibitors at the serotonin 5-HT2Areceptor.

Cariprazine hydrochloride is a novel atypical antipsychotic that has antagonistic effect on dopamine D3 receptor, dopamine D2 receptor and 5-hydroxytryptamine 2B receptor. It can be used for treating schizophrenia and bipolar I disorder.

The chemical name of cariprazine hydrochloride is 3-[4-[2-[4-(2,3-dichlorophenyl)piperazin-1-yl]ethyl]cyclohexyl]-1,1-dimethylurea;hydrochloride and its chemical structure is shown in the Formula I:

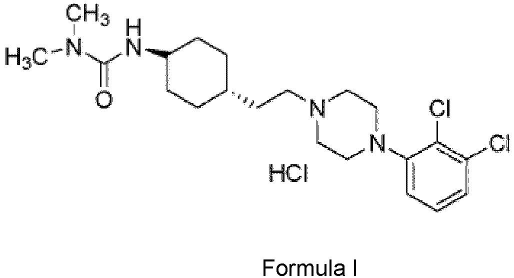

Cariprazine hydrochloride capsules are currently available on the market. This product is an oral preparation that should be taken daily to maintain its plasma concentration.

The patent EP4205745A1 disclose an aqueous suspension containing cariprazine, a preparation method and an application thereof. The pharmaceutical composition comprises: cariprazine pamoate solid particles, where a particle size of the cariprazine solid particles has a D(10) less than or equal to 30 microns, a D(50) less than or equal to 50 microns, and a D(90) less than or equal to 100 microns.

The patent CN117243955A disclose relates to the technical field of pharmaceutical preparations and discloses a cariprazine hydrochloride pharmaceutical composition and a capsule preparation containing the composition.

In the prior art documents, drug micronization is a commonly used technical measure to improve dissolution, but for cariprazine hydrochloride, micronized cariprazine hydrochloride has poor fluidity, is prone to static electricity, and is prone to agglomeration during the preparation process. It does not meet quality standards and is not easy to carry out large-scale production.

Therefore, the present invention focuses on selecting a suitable formulation to overcome the defects of cariprazine hydrochloride such as low solubility, low flowability and agglomeration, so as to ensure the stable quality of cariprazine hydrochloride capsules.

### Detailed Description of the Invention

The main object of the present invention is to a capsule formulation comprising cariprazine hydrochloride in a formulation providing good capsule filling and the desired dissolution profile and excellent pharmacotechnical properties, such as flowability and content uniformity.

Another object of the present invention is to provide a capsule formulation comprising cariprazine hydrochloride which is characterized by high stability.

Another object of the present invention is to provide a method for a capsule formulation comprising cariprazine hydrochloride prepared by simple, easy, time-saving and fast manufacturing methods.

As used herein, "bulk density" is the mass of particles of material divided by the total volume the particles occupy. The total volume includes particle volume, inter-particle void volume and internal pore volume. Bulk density is not an intrinsic property of a material; it can change depending on how the material is processed. The bulk density is expressed in grams per milliliter (g/mL) although the international unit is kilogram per cubic meter (1 g/mL=1000 kg/m³). It may also be expressed in grams per cubic centimeter (g/cm³).

Cariprazine hydrochloride ratio is used very low amount in formulations. Therefore, the use of other excipients in the formulation is quite high. This causes some problems such as flowability and capsule filling problems.

In order to create an ideal capsule that it does not cause any problems in swallowing, is of ideal size and easy, the excipients and their usage rates can be changed. The critical point here is the bulk density ratio of the final mixture. We saw that when the bulk density ratio was at least 0.5 g/cm³, capsule filling was done easily. Using a small amount of the active ingredient in the formulation did not cause any problems and the desired dissolution profile was achieved.

According to main embodiment, a capsule formulation comprises cariprazine hydrochloride and at least one filler and at least one lubricant, wherein the bulk density of this formulation before filling into capsules is at least 0.5 g/cm³ as measured by gravimetric determination of a measured volume.

This physical property of the formulation is very important for making the capsule that can be filled into suitable sized capsules for human consumption.

Suitable filler which are selected from the group comprising microcrystalline cellulose, sodium starch glycolate, lactose, lactose monohydrate, spray-dried lactose monohydrate, anhydrous lactose, mannitol, maltodextrin, dicalcium phosphate, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, polydextrose, polymethacrylates, sodium alginate, trehalose, polysorbate 80 or mixtures thereof.

According to this embodiment of the present invention, the amount of filler is between 80.0% and 98.0% by weight of the total formulation. Preferably, the amount of filler is between 90.0% and 96.0% by weight of the total formulation. The amount provides homogeneity.

The filler ratio is used in high proportions in the formulation. This causes problems in some capsule fillings. We have seen that filling into the capsule becomes easier especially when using fillers with a bulk density of 0.35 g/cm³ and above.

According to this embodiment of the present invention, this filler is microcrystalline cellulose, especially, MCC pH 302, which has a bulk density ratio higher than 0.35 g/cm³, is used.

According to this embodiment of the present invention, this filler is sodium starch glycolate which has a bulk density ratio higher than 0.35 g/cm³.

Suitable lubricants are selected from the group comprising anhydrous colloidal silicon dioxide, magnesium stearate, sodium stearyl fumarate, magnesium oxide, silicone dioxide, talc, polyethylene glycol, stearic acid, aluminum silicate, magnesium silicate, colloidal silica, or mixtures thereof.

According to an embodiment of the present invention, the lubricant is magnesium stearate.

According to this embodiment of the present invention, the amount of lubricant is between 0.01% and 5.0% by weight of the total formulation.

According to one embodiment of the invention, the capsule formulation further comprises binders.

Suitable binders are selected from the group comprising hydroxypropyl cellulose, corn starch, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 0.5% and 10.0% by weight of the total formulation. Preferably, the amount of binders is between 1.0% and 5.0% by weight of the total formulation.

According to this embodiment of the present invention, the binder is hydroxypropyl cellulose.

According to one embodiment of the present invention, the amount of cariprazine hydrochloride is between 0.1% and %10.0 by weight in the total formulation.

As used here in, 'particle size' means the cumulative volume size distribution as tested by any conventionally accepted method such as the laser diffraction method (i.e. Malvern analysis).

The term d (0.9) means, the size at which 90% by volume of the particles are finer. The term d (0.5) means, the size at which 50% by volume of the particles are finer.

According to one embodiment, a capsule formulation comprises cariprazine hydrochloride has a d (0.9) particle size between 30 µm and 1 µm.

According to one embodiment, cariprazine hydrochloride has a d (0.9) particle size between 30 µm and 2 µm, between 25 µm and 3 µm, between 20 µm and 3 µm, between 15 µm and 4 µm, between 10 µm and 1 µm.

According to an embodiment of the present invention, the capsule formulation comprises;
a) Cariprazine hydrochloride
b) Microcrystalline Cellulose
c) Hydroxypropyl cellulose
d) Magnesium stearate

According to an embodiment of the present invention, the capsule formulation comprises;
a) Cariprazine hydrochloride
b) Sodium starch glycolate
c) Magnesium stearate

According to an embodiment of the present invention , the capsule formulation is produced by direct compression method.

### Example 1; Capsule formulation

| **Ingredients** | **% by weight** | **% by weight** | **% by weight** | **% by weight** | **% by weight** |
|---|---|---|---|---|---|
| Cariprazine hydrochloride | 1.21 | 2.41 | 3.62 | 4.82 | 0.1-10 |
| Microcrystalline Cellulose pH 302 | 95.28 | 94.07 | 9.86 | 91.66 | 80-98 |
| Hydroxypropyl cellulose LXF | 2.78 | 2.78 | 2.78 | 2.78 | 0.5-10 |
| Magnesium stearate | 0.74 | 0.74 | 0.74 | 0.74 | 0.01-5 |
| **Total Weight** | 100 | 100 | 100 | 100 | 100 |

A process for example 1;
a) Mixing cariprazine hydrochloride and hydroxypropyl cellulose together and sieving through 850 microns,
b) Sieving of microcrystalline cellulose through 600 micron,
c) geometric mixing of the mixture in steps (a) and (b)
d) Sieving the mixture in step (c) through 850 micron sieve,
e) Sieving magnesium stearate through a 600 micron and adding magnesium stearate in step (d) and mixing,
f) Filling the mixture into capsules with an inner capsule weight of 135 mg.

### Example 2; Capsule Formulation

| **Ingredients** | **% by weight** |
|---|---|
| Cariprazine hydrochloride | 1.21 |
| Sodium starch glycolate | 98.05 |
| Magnesium stearate | 0.74 |
| **Total Weight** | 100 |

A process for example 2;
a) Sieving cariprazine hydrochloride and the half of sodium starch glycolate,
b) Sieving the remaining part of sodium starch glycolate,
c) Mixing the mixture at step (a) and at step (b)using the geometric mixing method,
d) Sieving the mixture,
e) Sieving magnesium stearate adding magnesium stearate in step (d) and mixing,
f) Filling the mixture into capsules with an inner capsule weight of 135 mg.

## Claims

1. A capsule formulation comprising cariprazine hydrochloride and at least one filler and at least one lubricant, wherein the bulk density of this formulation before filling into capsules is at least 0.5 g/cm³ as measured by gravimetric determination of a measured volume.

2. The capsule formulation according to claim 1, wherein filler which are selected from the group comprising microcrystalline cellulose, sodium starch glycolate, lactose, lactose monohydrate, spray-dried lactose monohydrate, anhydrous lactose, mannitol, maltodextrin, dicalcium phosphate, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, polydextrose, polymethacrylates, sodium alginate, trehalose, polysorbate 80 or mixtures thereof.

3. The capsule formulation according to claim 1, wherein the amount of filler is between 80.0% and 98.0% by weight of the total formulation.

4. The capsule formulation according to claim 1, wherein fillers with a bulk density of 0.35 g/cm³ and above is used.

5. The capsule formulation according to claim 2, wherein this filler is microcrystalline cellulose.

6. The capsule formulation according to claim 2, wherein this filler is sodium starch glycolate.

7. The capsule formulation according to claim 1, wherein lubricants are selected from the group comprising anhydrous colloidal silicon dioxide, magnesium stearate, sodium stearyl fumarate, magnesium oxide, silicone dioxide, talc, polyethylene glycol, stearic acid, aluminum silicate, magnesium silicate, colloidal silica, or mixtures thereof.

8. The capsule formulation according to claim 7, wherein the lubricant is magnesium stearate.

9. The capsule formulation according to any preceding claim, wherein further comprises binders.

10. The capsule formulation according to claim 9, wherein binders are selected from the group comprising hydroxypropyl cellulose, corn starch, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

11. The capsule formulation according to claim 10, wherein, the amount of binders is between 0.5% and 10.0% by weight of the total formulation.

12. The capsule formulation according to claim 10, wherein the binder is hydroxypropyl cellulose.

13. The capsule formulation according to any preceding claim, wherein cariprazine hydrochloride has a d (0.9) particle size between 30 µm and 1 µm.
